# EUROPEAN PATENT SPECIFICATION

(11) **EP 0 515 323 B1**
(45) Date of publication and mention of the grant of the patent: **22.01.1997**
(21) Application number: 92830232.2
(22) Date of filing: 18.05.1992
(51) Int. Cl.: G01N 33/78, G01N 33/559

(54) **Method of thyroglobulin dosage and kit for the use thereof**
Verfahren zur Bestimmung von Thyroglobulin und Kit zu dessen Gebrauch
Procédé pour le dosage de la thyroglobuline et kit nécessaire pour sa mise en oeuvre

(30) Priority: 20.05.1991 IT RM910344
(43) Date of publication of application: 25.11.1992
(73) Proprietor: CONSIGLIO NAZIONALE DELLE RICERCHE, I-00195 Roma (IT)
(72) Inventor: Corvo, Luciano Istituto di Medicina Sperimentale, I-00137 Rome (IT); Salabè, Giovanni Battista, I-00137 Rome (IT)
(74) Representative: de Simone, Domenico

(56) References cited:
- EP-A- 0 396 342
- PROCEEDINGS OF THE SERONO SYMPOSIA vol. 33, 1980, pages 147 - 152; S.FONTANA ET AL.: 'Thyroglobulin quantitation by a modified hemolytic radial immunodiffusion assay.'
- THE JOURNAL OF INTERNATIONAL MEDICAL RESEARCH vol. 18, 1990, pages 253 - 255; M.BAYRAKTAR ET AL.: 'A Preliminary Report of Thyroglobulin Release after Fine Needle Aspiration Biopsy of Thyroid Nodules/

## Description

This invention relates to a method of thyroglobulin dosage.

More particularly, this invention relates to a method of thyroglobulin dosage which is suitable to determine thyroglobulin levels which are present in the thyroid gland and which is rapid, simple and accurate as well as of low cost for employment even in clinical laboratories not highly specialized, and by means of a kit.

The bibliographic references mentioned herein refer to the list of references attached to the end of the present disclosure.

Human thyroglobulin (hTg) is the organospecific protein of the thyroid gland, which protein is secreted by utricular cells and becomes settled in the colloid. The synthesis, the metabolism, the primary immunochemical structure and the function in the genesis of hormones have all been widely investigated (1-5).

The hTg concentration per gram of fresh tissue is between 20 and 40 mg. Such value can vary in different conditions of altered thyroidal function. The concentration of hTg decreases in Basedow's disease, in the treatment with antithyroid, in the hot nodule of thyroid, in goitre caused by alteration in the genesis of hormones, whereas, it increases in the case of colloid goitre and following the treatment with iodine (6, 7).

In thyroidal neoplasms, a progressive reduction of protein with respect to the differentiation degree of neoplasms occurs: said protein is present in adenomas and in the endemic and in sporadic multinodular goitre, is remarkably reduced in the case of follicular and papillary cancer, is absent in anaplastic neoplasms, in thyroidal lymphoma and in medullary carcinoma (8).

The thyroidal nodular pathology is a very frequent occurrence generally among people, but it is impossible to make a precise and single percentage estimate because of the wide geographical variability and the multiplicity of the manifestations of the disease, especially in the zones of goitre endemic disease. However, it is possible to estimate approximately that a percentage of 5-15 % of the general people is affected with goitre and/or with the nodular pathology of thyroid (11).

Accordingly, the cytological test on the thyroidal matter aspirated by a needle has become widely spread in the clinical practice in the most recent years as a guide to the selection of a suitable therapeutical treatment (the medical or the surgical treatment) of the thyroidal nodule for judge about the alternatives of a colloid nodule or of a neoplasm in its different differentiation steps.

The cytological test carried out on the utricular cells of matter-aspirated through a needle can however give doubts about the malignant of the benign nature of the thyroidal tumefaction. The quantitative determination of hTg, which protein represents the main specific-constituent of the colloid can be of great help in formulating and integrating the cytological diagnosis, so allowing the cytological criteria to be supported by the quantitative determination of a cellular differentiation marker.

S. Fontana et al. (Proceedings of the Serono Symposia, vol. 33, 1980, 147-152) describe the thyroglobulin quantitation by a hemolytic radial immmodiffusion assay, able to detect as little as 60 to 80 pg thyroglobulin. EP-A-396342 describes a radial immmodiffusion technique in order to assess the concentration of an analyte. M. Bayraktar et al. (The Journal of International Medical Research, vol. 18, 1990, 253-255) describe the effect of needle aspiration of thyroid nodules on serum thyroglobulin concentration.

The dosage of thyroglobulin in the thyroidal matter aspirated through a needle is not carried out currently because of the fact that the available procedures (RIA, IRMA, ELISA). developed for determine quantitatively the hTg concentration in human serum can determine amounts of hTg of the order of magnitude of ng so that they are quite unsuitable for the application to the quantitative determination of the intrathyroid concentration of hTg which is, on the contrary, of the order of mg.

Accordingly, it is evident that there is the need for a simple, accurate and rapid procedure for the quantitative determination of thyroglobulin in thyroidal cellular matter sucked through needles, of the order of magnitude of mg without the need for highly specialized laboratories so that said procedure can be employed for clinical and diagnosis aims.

The authors of this invention have found that an immuno-diffusion procedure satisfies such characteristics so that it can be exploited for the quantitative determination of thyroglobulin in thyroidal matter sucked through needles, in a rapid and accurate way without the need for employment of radioisotopic material.

Accordingly, it is an object of the invention to provide a method for the diagnosis of thyroid gland disorder involving the quantitative determination of thyroglobulin therein, in which thyroglobulin in a thyroidal sample drawn by aspiration is quantitatively determined. Preferably the quantitative determination of thyroglobulin is obtained by means of immmodiffusion techniques. More preferably immunodiffusion techniques comprise simple radial immunodiffusion.

In a preferred embodiment the method comprises the following steps:
- preparing an inert gel containing a ligand capable of ligating thyroglobulin;
- contacting the thyroidal sample with said gel;
- incubating the same;
- quantitatively determining the ligandthyroglobulin reaction.

Preferably the ligand comprises anti-thyroglobulin immune sera or anti-thyroglobulin purified antibodies.

Preferably the inert gel comprises agarose at a concentration in the range from 0.8% to 2.0%. More preferably the agarose is dissolved in a solution containing NaCl and a phosphate buffer. Most preferably NaCl is at 0.15 M concentration and the solution comprises sodium glutamate and chlorhexidine.

Preferably the inert gel is poured in the liquid state into the space between two glass plates suitably sealed and then it is allowed to solidify. More preferably one of said two glass plates is covered with a material that improves the adhesion of said gel to said glass plate.

According to an embodiment of the invention the contacting the thyroidal sample with the gel occurs through aspiration by means of a needle of a portion of said inert gel and the formation of a circular well into which said thyroidal sample is introduced.

Preferably the incubation is carried out in a humid chamber for a time between 2 hours and 60 hours. More preferably the temperature of the humid chamber is in the range between 20°C and 27°C and the time is between 24 hours and 50 hours.

Preferably the reaction between the ligand and thyroglobulin is a precipitation reaction and said quantitative determination is carried out by measuring at least one diameter of precipitation rings. More preferably precipitation rings are colored by means of protein dyes after drying said inert gel.

According to another aspect of the invention the thyroidal sample contains thyroglobulin at a concentration between 0.5 and. 4.0 mg/ml or is diluted for obtaining said concentration.

This invention will be disclosed in the following with reference to some application examples which are to be thought of as illustrative but not limitative examples.

### Example 1

### A procedure for the quantitative determination of thyroglobulin for simple radial immunodiffusion

Immunodiffusion techniques are disclosed in (12).

The aspiration by means of the needle is carried out employing a device for aspiration cytological matter (Cameco, Sweden) with a 20 ml syringe and 21 G x 1-1/2, 0.8 x 40 mm needle.

The material so aspirated can consist of:

a) a few drops of hematic liquid; b) cystic liquid in variable amounts (larger than 20 ml), brown-colored or of yellow- or hematic-colour. In case a), a fraction of the volume (30 µl) is diluted in 0.15 M NaCl, 1+1 and then such volume is centrifuged within polyethylene tubes (29 x 6 mm), 2' at 1,000 x g; the supernatant is employed for the quantitative determination of hTg, the portion settled down is carefully suspended and then it was smeared for carrying out the cytological examination. In case b), the cystic liquid is centrifuged (1,500 x g x 10') and in a way similar to the case a), the supernatant was employed for the quantitative determination of hTg while the sedimented portion was employed for the cytological examination.

1.5 g of agarose was dissolved by heating into 100 ml of a saline solution (0.15 M NaCl) that had been buffered with 0.05 M phosphate buffer containing sodium glutamate 0.24 M and 0.005 % chlorhexidine. The volume is distributed among 10 ml tubes and they are stored at +4°C.

Rabbit human anti-thyroglobulin serum is prepared by emulsifying 5 mg of hTg in 1 ml of Freund complete adjuvant and injecting the emulsion into the adipose cushions of the paws of the animal. One month after, a booster injection is performed following the same operations. After 15 days, the animal was bled by cardica puncturing, the antibody is absorbed with normal human serum and the concentration was determined by plotting the curve of precipitins. A titre of 10 mg of precipitated hTg was obtained from the precipitin curve at the equivalence per ml of serum.

The hTg is prepared from a raw extract of a colloid goitre through salting and successive chromatography on a superfine Sephadex® G 200 column (0.05 M phosphate buffer; pH 7.4). The purity check gives a single band in the SDS electrophoresis on polyacrylamide gel (8-10).

10 ml of agar is dissolved at 80°C on a water bath and then the solution is allowed to cool; when the agar is at 56°C, 1 ml of the rabbit anti-hTg is added, then the mixture is rapidly put on a magnetic stirrer, so as to obtain a complete mixing, then the agar-anti-serum is distributed while it is in the molten state by means of a pipette into the mold.

The mold is obtained by putting the two glass plates (7 x 15 cm) near to one another after cleaning them accurately, the two plates being separate by a U-shaped intermediate member of a plastic material and being kept together by means of metallic claws. One of said two plates is previously prepared by covering the same with a Gel Bond lamina (Pharmacia).

The gel-antiserum mixture is allowed to cool fo one hour down to room temperature, then the plate not treated with Gel Bond is removed by causing it to slide gently. The u-shaped plastic intermediate member is also removed. By puncturing the gel with an aspiration needle of 2.5 mm supplied with a telescopic device, by means of gentle aspiration (water pump vacuum) two rows of 5 wells are obtained, the diameter of each well being of 2.5 mm. 0.01 ml of a solution containing increasing amount of hTg (0.005-0.04 mg) are put into 5 wells by means of an Eppendorf pipette or similar, taking care of changing the tip of the pipettes at each deposition; the various samples to be examined are put into the other wells. The plates are then left for 48 hours in a humid chamber at room temperature. At the end of the incubation time, the diameter of the precipitation rings is read by means of the suitable plate-reader rule. Two diameters at right angles are measured, and the average value is taken. The two diameters are not to differ from one another by more than 5 %. The reading can also be carried out more accurately on the colored rings, in addition to the reading carried out on the cooled material. To that aim, the plates are washed in water for some hours and then they are dried in an oven at 100°C for 30 minutes. Proteins are the colored by employing the usual protein dyes (bromphenol blue, Coomassie, Ponceau Red). The reference curve is prepared by taking the hTg concentration as the abscissas and the diameter of the rings in mm as the ordinates.

Amounts of hTg from 0.5 to 4 mg/ml of sample can be determined by this procedure.

In the case of more concentrated samples, it is suitable to realize proper dilutions.

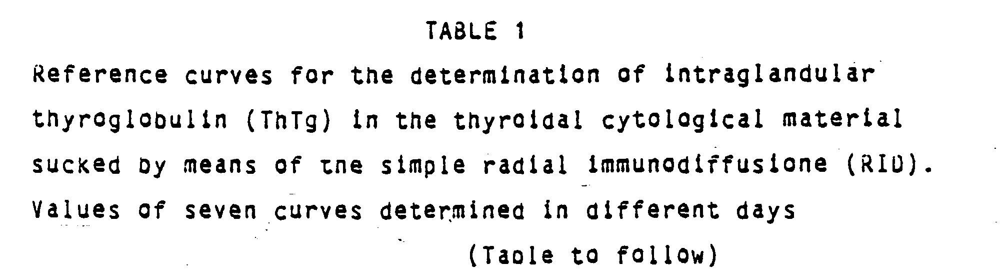

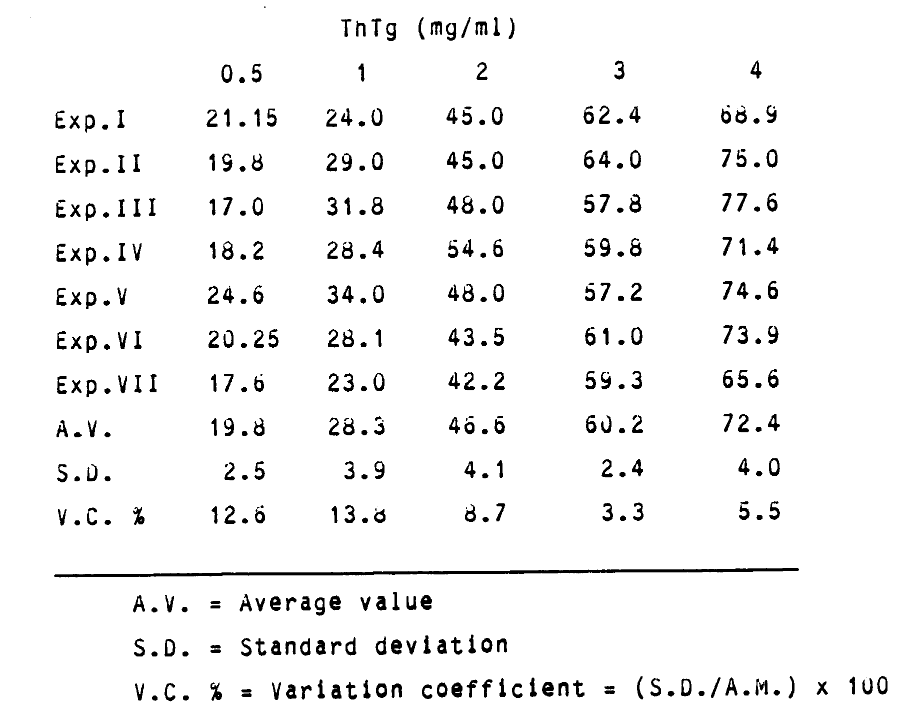

### Example 2

### Results obtained employing the method of quantitative determination of thyroglobulin

The results of seven reference curves are illustrated in Table 1. The variability of the different experiments has reached its maximum values of 13 % at the lowest points of the curve whereas it has reached maximum values of 8 % and minimum values of 3 % in samples having higher concentrations.

The comparative estimate between the simple radial immunodiffusion (RID) and the method RIA, carried out employing commercial kits on a limited number of samples, has shown an optimal correlation (r = 0.93, p less than 0.01).

The intrathyroidal concentration of ThTg determined by the RID technique in the cytological material sampled through suction of 54 thyroidal tumefactions is reported in Table 2.

**Table 2**

| Intrathyroidal concentration of thyroglobulin (ThTg) determined through the simple radial immunodiffusion technique in the cytological material sampled through suction of 54 thyroidal tumefactions with different cytological or clinical diagnosis. | | | |
|---|---|---|---|
| Cytological or clinical laboratory diagnosis | No. of cases | ThTg (mg/ml) | ThTg (mg/ml) (very high values) |
| without typical elements | 6 | 1.42±0.93 | - |
| nodular hyperplasia | 8 | 1.32±0.5 | 5-16-40 |
| hot nodule | 5 | nd or < 0.5 | -1.3- |
| follicular neoplasia | 3 | nd or < 0.5 | - |
| lymphocytic chronical thyroiditis | 5 | nd or < 0.5 | - |
| Graves' disease | 2 | nd or < 0.5 | - |
| denatured pseudocyst | 11 | 1.58±0.69 | 5-5-6.5-18.5 |
| serous pseudocyst | 14 | nd or < 0.5 | - |
| nd = Non determinable | | | |

In nodular hyperplasia, the average concentration of ThTg showed to be 1.30 ± 0.5 mg/ml, however in three out of eight cases, which are not included in the calculations of the average value, the concentration of ThTg turned out to be definitely higher because it was necessary to dilute the sample for reading the same out of the reference curve. Concentrations similar to those of the hyperplastic nodule were observed in denatured pseudocyst (1.58 ± 0.69 mg/ml). Such pseudocyst can be considered as a hyperplastic nodule gone towards necrosis and fluidification. In such group also some very high values are reported separately. In such cases, colloidocystic degeneration has occurred. It is interesting to stress that in a group of six amounts of cytological matter extracted by aspiration, in which the typical thyroid elements could not be evidenced (edges of utricular cells or thyroidal follicles) it was possible to determine quantitatively a concentration of ThTg comparable to that observed in the hyperplastic nodule. This observation puts into evidence a further advantage of such method because even in the absence of typical cellular members it supplies an organ diagnosis element as well as a judgement about the nature of the tumefaction.

In the other groups of thyroidal pathologies classified according to cytological criteria (chronic lymphocytic thyroiditis, follicular neoplasia) or according to clinical laboratory criteria (hot nodule, Graves' disease) the concentration of ThTg turned out to be absent or present at concentrations of 0.5 mg/ml. This result is in good agreement with what is already known from histological and biochemical studies in which it is shown that in the case of Graves' disease, in the hot nodule, in adenoma and in thyroidal carcinoma the colloid in which the ThTg deposits is very scarce, as well as in lymphocytic thyroiditis in which the follicular tissue is substituted with the lymphatic infiltration.

Accordingly, in the presence of a cytological situation of "follicular hyperplasia" or of suspected "lymphocytic thyroiditis" the quantitative determination of ThTg can supply a useful element of diagnostic integration. For instance, when in doubt between "chronic lymphocytic thyroi ditis" and focal thyroiditis in nodular hyperplasia" the quantitative determination of ThTg can give indications towards the diagnosis of chronic lymphocytic thyroiditis (ThTg of low concentration or even absent) or towards the diagnosis of nodular hyperplasia (high concentration of ThTg). In a similar way, in the case of a cytological matter extracted by suction which is rich of cells, when in doubt between hyperplasia or nodular neoplasia, the determination of ThTg can supply indications towards the diagnosis of hyperplasia if high values of ThTg are obtained, or towards the diagnosis of neoplasia if ThTg is absent or is present at low concentrations.

### BIBLIOGRAPHIC REFERENCES

1) Biochimie 72, 9.1989. The whole volume.
2) RAWTICH A.B, LITWER M.R., GREGG J., TURNER C.D., ROUSE J.B., HAMILTON J.W.: The Isolation of Identical Thyroxine Containing Amino Acids Sequences from Bovine, Ovine and Porcine Thyroglobulins. Biochem. Biophys. Res. Commun. 118, 423-429, 1984
3) MERCKEN L., SIMONS M.J., SWILLENS S., MASSAER M., VASSART G.: Primary structure of bovine thyroglobulin deduced from the sequence of its 8431 - base complementary DNA. Natura (Lond.) 316, 647-651, 1985.
4) T. HOTTA, I. ISHII, H. ISHIHARA, S. TEJIMA, O. TARUTANI, N. TAKAHASHI: "Comparative study of oligosaccharides of human Thyroglobulins obtained from normal subjects and patients with various diseses". J. Appl. Biochemistry 7, 98-103, 1985.
5) GARTNER R., BECHTNER G., GREIL W., HORN K., PICKARDT C. R.: "Characterization of microheterogeneity of human thyroglobulin from different thyroid disorders". Acta Endocrinologica 109, 76-82, 1985.
6) SALABE' G.B., PAPA C.: Proteine solubili tiroidee; studio elettroforetico di estratti di tiroide normale e patologica, Folia Endocr. 17, 372, 1964.
7) SALABE' G.B., BASCHIERI L., TONELLI S.: Studio delle proteine solubili della tiroide di soggetti trattati con soluzione iodio-iodurata e con tiuracilici. Folia Endocr. 17, 293, 1964.
8) OLIVIERI A., MARZULLO A;, SALABE' G.B., FAZZINI C., GILARDI E. , CARTA S., Isoelectric pattern of thyroglobulin and antithyreoglobulin antibodies. Thyroidology. in print.
9) DERRINE Y., MICHEL R., PEDERSEN K.O., ROCHE J.: Recherches sur la preparation et les propriétés de la thyréoglobuline pure. Biochim. Biphys. Acta 3, 346, 1949.
10) SALVATORE G., SALVATORE M., CAHUMANN H.J., ROBBINS J.: Separation of Thyroidal Iodoproteins and Purification of Thyroglobulin by Gel Filtration and Density Gradient Centrifugation. J. Biol. CHEM. 239, 3267-3274, 1964.
11) TUNBRIDGE W.M.G., EVERED D.C., HALL R., APPLETON D., BRIWIS M., CLARK F., GRIMLEY EVANS J. YOUNG E., BIRD T., SMITH P.A.; The Sectrum of thyroid disease in a community: The Whickam survey Clinical Endocr. 7, 481-493, 1977.
12) CROWLE A.J.; Immunodiffusion, second edition, Academic Press New York and London, 1973.

## Claims

1. A method for the diagnosis of thyroid gland disorder involving the quantitative determination of thyroglobulin therein, characterized in that thyroglobulin in a thyroidal sample which has been drawn by aspiration is quantitatively determined.

2. A method for the diagnosis of thyroid gland disorder according to claim 1 wherein the quantitative determination of thyroglobulin is obtained by means of immunodiffusion techniques.

3. A method for the diagnosis of thyroid gland disorder according to claim 2 wherein said immunodiffusion techniques comprise the simple radial immunodiffusion.

4. A method for the diagnosis of thyroid gland disorder according to claim 3, said method comprising the following steps:
- preparing an inert gel containing a ligand capable of ligating thyroglobulin;
- contacting the thyroidal sample with said gel;
- incubating the same;
- quantitatively determining the ligand-thyroglobulin reaction.

5. A method for the diagnosis of thyroid gland disorder according to claim 4, wherein said ligand comprises anti-thyroglobulin immune sera or anti-thyroglobulin purified antibodies.

6. A method for the diagnosis of thyroid gland disorder according to claim 4 or 5,wherein said inert gel comprises agarose at a concentration in the range from 0.8% to 2.0%.

7. A method for the diagnosis of thyroid gland disorder according to claim 6, wherein said agarose is dissolved in a solution containing NaCl and a phosphate buffer.

8. A method for the diagnosis of thyroid gland disorder according to claim 7, wherein said NaCl is at 0.15 M concentration and said solution comprises sodium glutamate and chlorhexidine.

9. A method for the diagnosis of thyroid gland disorder according to any one of the preceding claims 4-8 wherein said inert gel is poured in the liquid state into the space between two glass plates suitably sealed and then it is allowed to solidify.

10. A method for the diagnosis of thyroid gland disorder according to claim 9, wherein one of said two glass plates is covered with a material that improves the adhesion of said gel to said glass plate.

11. A method for the diagnosis of thyroid gland disorder according to any one of the preceding claims 4-10 wherein said contacting occurs through aspiration by means of a needle of a portion of said inert gel and the formation of a circular well into which said thyroidal sample is introduced.

12. A method for the diagnosis of thyroid gland disorder according to any one of the preceding claims 4-11 wherein said incubation is carried out in a humid chamber for a time between 2 hours and 60 hours.

13. A method for the diagnosis of thyroid gland disorder according to claim 12, wherein the temperature of said humid chamber is in the range between 20°C and 27°C and said time is between 24 hours and 50 hours.

14. A method for the diagnosis of thyroid gland disorder according to any one of the preceding claims 4-13 wherein said reaction between the ligand and thyroglobulin is a precipitation reaction and said quantitative determination is carried out by measuring at least one diameter of the precipitation rings.

15. A method for the diagnosis of thyroid gland disorder according to claim 14, wherein said precipitation rings are colored by means of protein dyes after drying said inert gel.

16. A method for the diagnosis of thyroid gland disorder according to any one of the preceding claims wherein said thyroidal sample contains thyroglobulin at a concentration between 0.5 and. 4.0 mg/ml or is diluted for obtaining said concentration.

## Patentansprüche

1. Verfahren zur Diagnose von Schildrüsen-Krankheiten mit der mengenmässigen Bestimmung des Zugehörigen Thyreoglobulins, dadurch gekennzeichnet, dass das Thyreoglobulin in einer durch Aspiration entnomener Schildrüsenprobe mengenmässig bestimmt wird.

2. Verfahren zur Diagnose von Schildrüsen-Krankheiten nach Anspruch 1, worin die mengenmässige Bestimmung von Thyreoglobulin durch Immunodiffusion-Methoden erhalten wird.

3. Verfahren zur Diagnose von Schildrüsen-Krankheiten nach Anspruch 2, worin die vorgenannten Immunodiffusionsmethoden die einfache radiale Immunodiffusion umfassen.

4. Verfahren zur Diagnose von Schildrüsen-Krankheiten nach Anspruch 3, worin das vorgenannte Verfahren folgende Verfahrensschritte aufweist:
- Vorbereitung eines inerten, einen Liganden enthaltenden Gels;
- Zusammenbringen der Schildrüsenproben mit dem vorgenannten Gel;
- Inkubation derselben;
- mengenmässige Bestimmung der Ligand-Thyreoglobulin-Reaktion.

5. Verfahren zur Diagnose von Schildrüsen-Krankheiten nach Anspruch 4, worin der vorgenannte Ligand Antithyreoglobulin-Immunoserum oder Antithyreoglobulin gereinigte Antikörper enthält.

6. Verfahren zur Diagnose von Schildrüsen-Krankheiten nach Anspruch 4 oder 5, worin die vorgenannten inerte Gel Agarose in einer Konzentration im Bereich von 0,8% bis 2,0% enthält.

7. Verfahren zur Diagnose von Schildrüsen-Krankheiten nach Anspruch 6, worin das vorgenannte Agarose in einer NaCl und eine Phosphat-Puffer enthaltenden Lösung gelöst ist.

8. Verfahren zur Diagnose von Schildrüsen-Krankheiten nach Anspruch 7, worin das vorgenannte NaCl in einer Konzentration von 0.15 M vorliegt und die vorgenannte Lösung Natriumglutamate und Chlorhexidin enthält.

9. Verfahren zur Diagnose von Schildrüsen-Krankheiten nach je einem der vorhergehenden Ansprüche 4 bis 8, worin das vorgenannte inerte Gel im flüssigen Zustand im Raum zwischen zwei entsprechend gedichteten Glasplatten eingegossen und aushärten gelassen wird.

10. Verfahren zur Diagnose von Schildrüsen-Krankheiten nach Anspruch 9, worin eine der vorgenannten Glasplatten mit einem material bedeckt ist, das das Anhaften des vorgenannten Gels an die vorgenannte Glasplatte verstärkt.

11. Verfahren zur Diagnose von Schildrüsen-Krankheiten nach je einem der vorhergehenden Ansprüche 4 bis 10, worin das vorgenannte Zusammenbringen durch Ansaugen durch eine Nadel eines Teiles des vorgenannten inerten Gels und durch Bildung eines kreisförmigen Schachtes, in welchen die vorgenannte Schildrüsenpobe eingeführt ist, durchgefuehrt ist.

12. Verfahren zur Diagnose von Schildrüsen-Krankheiten nach je einem der vorhergehenden Ansprüche 4 bis 11, worin die vorgenannte Inkubation in einer nassen Kammer für eine Zeitdauer von 2 bis 60 Stunden durchgeführt wird.

13. Verfahren zur Diagnose von Schildrüsen-Krankheiten nach Anspruch 12, worin die Temperatur der vorgenannte nassen Kammer im Bereich von 20°C bis 60°C und die vorgenannte Zeitdauer im Bereich von 24 bis 50 Stunden gehalten werden.

14. Verfahren zur Diagnose von Schildrüsen-Krankheiten nach je einem der vorhergehenden Ansprüche 4 bis 13, worin die vorgenannte Reaktion zwischen dem Liganden und dem Thyreoglobulin eine Präzipitations-reaktion ist und die vorgenannte mengenmässige Bestimmung durch Ausmessung mindestens eines Durchmessers der Präzipitationsringe vorgenommen wird.

15. Verfahren zur Diagnose von Schildrüsen-Krankheiten nach Anspruch 14, worin die vorgenannten Präzipitationsringe durch Proteinfarben nach Trocknung des vorgenannten inerten Gels geförbt werden.

16. Verfahren zur Diagnose von Schildrüsen-Krankheiten nach je einem vorhergehende Ansprüche, worin das Schildrüsenprobe Thyreoglobulin in einer Konzentration zwischen 0,5 und 4,0 mg/ml enthält oder zur Erhaltung dieser Konzentration verdünt wird.

## Revendications

1. Procédé pour le diagnostic de maladie de la thyroideglande comprenant la détermination quantitative de thyroideglobuline contenue dans la même, caractérisé en ce que ladite thyroideglobuline est déterminée quantitativement dans un échantillon de thyroide prélevé par aspiration.

2. Procédé pour le diagnostic de maladie de la thyroideglande selon la revendication 1, dans lequel la détermination quantitative de thyroideglobuline est obtenue au moyen des techniques d'immunodiffusion.

3. Procédé pour le diagnostic de maladie de la thyroideglande selon la revendication 2, dans lequel lesdites techniques comprendent une immunodiffusion radiale simple.

4. Procédé pour le diagnostic de maladie de la thyroideglande selon la revendication 3, ledit procédé comprenant les stades suivants:
- préparation d'un gel inerte contenant un liant capable de lier la thyroideglobuline;
- mise en contact dudit échantillon de thyroide avec ledit gel;
- incubation de la même;
- determination quantitative de la réaction liant-thyroideglobuline.

5. Procédé pour le diagnostic de maladie de la thyroideglande selon la revendication 4, dans lequel ledit liant comprende immunsérum d'anti-thyreoglobuline ou anticorps depurés de thyreoglobuline.

6. Procédé pour le diagnostic de maladie de la thyroideglande selon la revendication 4 ou 5, dans lequel ledit gel inerte comprende agarose dans un concentration entre 0,8% e 2,0%.

7. Procédé pour le diagnostic de maladie de la thyroideglande selon la revendication 6, dans lequel ledit agarose est dissour dans une solution contenant NaCI et un tampon de phosphate.

8. Procédé pour le diagnostic de maladie de la thyroideglande selon la revendication 7, dans lequel ledit NaCI est à concentration de 0,15 M et ladite solution comprende glutamate de sodium et chlorhexide.

9. Procédé pour le diagnostic de maladie de la thyroideglande selon une quelconque des revendications précédentes 4 à 8, dans lequel ledit gel inerte est versé à l'état liquide dans l'espace entre deux plaques de verre fermées de façon étanche et laissé solidifier.

10. Procédé pour le diagnostic de maladie de la thyroideglande selon la revendication 9, dans lequel une de ces deux plaque de verre est recouvert avec un matériel qui meuilleur l'adhesion entre ledit gel et ladite plaque de verre.

11. Procédé pour le diagnostic de maladie de la thyroideglande selon une quelconque des revendications précédentes 4 à 10, dans lequel ladite mise en contact a lieu par aspiration au moyen d'une aiguille d'une portion dudit gel inerte et par formation d'un puits circulaire, dans lequel est introduit ledit échantillon de thyroide.

12. Procédé pour le diagnostic de maladie de la thyroideglande selon la revendications 4 à 11, dans lequel ladite incubation est réalisée dans une chambre humide pendant un temps entre 2 et 60 heures.

13. Procédé pour le diagnostic de maladie de la thyroideglande selon la revendication 12, dans lequel la température de ladite chambre humide est compris entre 20°C et 27° et ledit temps est compris entre 24 et 50 heures.

14. Procédé pour le diagnostic de maladie de la thyroideglande selon une quelconque des revendications précédentes 4 à 13, dans lequel ladite réaction entre le liant et la thyreoglobuline est une réaction de précipitation et ladite détermination quantitative est réalisée par mesurage au moin d'un diamétre des anneaux de précipitation.

15. Procédé pour le diagnostic de maladie de la thyroideglande selon la revendication 14, dans lequel lesdits anneaux de précipitation sont colorés au moyen de couleur à protéine après le séchage dudit gel inerte.

16. Procédé pour le diagnostic de maladie de la thyroideglande selon une quelconque des revendications précédentes, dans lequel l'échantillon de thyroide contient la thyreoglobuline à une concentration entre 0,5 et 4,0 mg/ml ou est dilué pour obtenir ladite concentration.
